# EUROPEAN PATENT APPLICATION

(11) **EP 4 785 878 A1**
(43) Date of publication of application: **05.08.2026**
(21) Application number: 24884257.7
(22) Date of filing: 10.09.2024
(51) Int. Cl.: A61B 17/072

(54) **SURGICAL INSTRUMENT**

(30) Priority: 30.10.2023 CN 202311426233
(71) Applicant: Fulbright Medical Inc., Wuxi, Jiangsu 214437 (CN)
(72) Inventor: SUN, Baofeng, Wuxi, Jiangsu 214437 (CN); SHI, Longxia, Wuxi, Jiangsu 214437 (CN); HU, Jie, Wuxi, Jiangsu 214437 (CN)
(74) Representative: Maiwald GmbH
(86) International application number: PCT/CN2024/117858
(87) International publication number: WO 2025/092250

(57) **Abstract**

Embodiments of the present disclosure relate to a surgical instrument, comprising an end effector and a cannula assembly. The cannula assembly comprises a pusher. The end effector comprises: a jaw assembly, comprising a staple cartridge base, the staple cartridge base having a limiting structure; and a staple cartridge assembly, detachably mounted in the staple cartridge base, the staple cartridge assembly comprising a staple pushing plate. The pusher is provided with a cutter lifting portion, and the staple pushing plate is provided with a support structure; when the staple cartridge assembly is mounted in the staple cartridge base and the staple pushing plate is located at a proximal side position, the cutter lifting portion is supported by the support structure, and in response to an operation of a user, the pusher crosses a limiting structure to move towards a distal side to mate with the staple pushing plate, so as to drive the staple pushing plate to move towards the distal side; and when the staple cartridge assembly is not mounted in the staple cartridge base or the staple pushing plate is located at a distal side position, in response to an operation of the user, the pusher is blocked by the limiting structure when moving towards the distal side. The embodiments of the present disclosure solve the problem that there is a risk of damaging a human body when a staple cartridge assembly which has been used is mounted in a surgical instrument provided with a staple cartridge integrated with a cutter or no staple cartridge assembly is mounted therein.

## Description

This application claims priority to Chinese Patent Application No. 202311426233.3 filed on October 30, 2023, the entire disclosure content of which is incorporated herein by reference as a part of the present application.

### TECHNICAL FIELD

Embodiments of the present disclosure relate to a surgical instrument.

### BACKGROUND

Surgical instruments play a pivotal role in clinical surgery, and a cutting blade is an indispensable and important component of a surgical instrument. The distal end of a surgical instrument includes a replaceable cartridge assembly, which is a disposable consumable.

To avoid wear and poor cutting effect caused by the repeated use of a cutting blade, a cartridge assembly integrated with a cutting blade has been developed. When the cartridge assembly is not mounted on a surgical instrument, the cutting blade is hidden in a cartridge body to prevent the sharp cutting edge from injuring a user; when the cartridge assembly is mounted on a surgical instrument for use, the cutting edge of the cutting blade extends out of the surface of the cartridge body, i.e., blade extension, so as to cut tissue during surgery.

When an unused cartridge assembly is mounted in a jaw assembly, the cutting blade moves forward until a full cutting stroke is achieved (cutting and suturing). However, when the jaw assembly is mounted with a used cartridge assembly or no cartridge assembly at all, the surgical instrument is in an empty cartridge state. When the cutting blade moves forward, only cutting without suturing will be performed, and the surgical instrument should identify this state to further prevent firing and avoid injuring the human body. In some cases, however, the empty cartridge protection structure is only applicable to traditional cutting blade assemblies and not to cartridges with an integrated cutting blade, resulting in a risk of injury to the human body when a surgical instrument with cartridge assembly integrated with a cutting blade is mounted with a used cartridge assembly or no cartridge assembly at all.

### SUMMARY

The surgical instrument provided by the embodiments of the present disclosure solves the problem that a surgical instrument with a cartridge integrated with a cutting blade has a risk of injuring the human body when a used cartridge assembly or no cartridge assembly is mounted.

An embodiment of the present disclosure provides a surgical instrument, includes an end effector and a sleeve assembly, the sleeve assembly includes a pusher, the end effector includes:
a jaw assembly includes a cartridge holder and an anvil pivotally connected to the cartridge holder; the cartridge holder being provided with a limiting structure;
a cartridge assembly detachably mounted in the cartridge holder, the cartridge assembly includes a pusher plate and a cutting blade movably connected to the pusher plate;
a blade lifting portion is provided at a bottom of the pusher, and a support structure is provided at a bottom of the pusher plate; when the cartridge assembly is mounted in the cartridge holder and the pusher plate is at a proximal position, the blade lifting portion is supported by the support structure, and the end effector is in a first state; in the first state, in response to a user's operation, the pusher passes over the limiting structure and moves distally to be engaged with the pusher plate, thereby driving the pusher plate to move distally;
when the cartridge assembly is not mounted in the cartridge holder or the pusher plate is at a distal position, the blade lifting portion is suspended within the cartridge holder, and the end effector is in a second state; in the second state, in response to a user's operation, the pusher is blocked by the limiting structure when moving distally.

For example, in the surgical instrument provided in an embodiment of the present disclosure, the support structure includes a support groove formed at the bottom of the pusher plate, and the support groove includes a bottom wall; when the end effector is in the first state, the bottom wall of the support groove abuts against the blade lifting portion to support the blade lifting portion.

For example, in the surgical instrument provided in an embodiment of the present disclosure, the support groove further includes an inclined section, where the inclined section is located at a proximal side of the bottom wall and connected to the bottom wall, and a proximal side of the inclined section is lower and a distal side thereof is higher.

For example, in the surgical instrument provided in an embodiment of the present disclosure, the pusher plate is provided with a receiving groove, the cutting blade is received in the receiving groove, and the receiving groove is in communication with the support groove; when the end effector is in the first state, a portion of the blade lifting portion is received in the receiving groove and another portion of the blade lifting portion is received in the support groove; an upper end surface of the blade lifting portion abuts against the cutting blade to keep the cutting blade in a blade extended state; a lower end surface of the blade lifting portion abuts against the bottom wall of the support groove.

For example, in the surgical instrument provided in an embodiment of the present disclosure, the pusher includes a limiting portion; in the first state, the limiting portion is at a first height and the limiting portion is higher than the limiting structure to cause the pusher to pass over the limiting structure; in the second state, the limiting portion is at a second height, and the limiting structure blocks the limiting portion to block the pusher; the second height is lower than the first height.

For example, in the surgical instrument provided in an embodiment of the present disclosure, the limiting structure is a groove, and the groove includes a stopping wall; when the pusher is at the first height, the limiting portion is higher than an upper edge of the stopping wall and located outside the groove; when the pusher is at the second height, the limiting portion is lower than the upper edge of the stopping wall and located inside the groove.

For example, in the surgical instrument provided in an embodiment of the present disclosure, the cartridge holder further includes a stopping protrusion, and the stopping protrusion is located at a distal side of the stopping wall and is higher than the upper edge of the stopping wall.

For example, in the surgical instrument provided in an embodiment of the present disclosure, the cartridge holder further includes an initial surface, and the initial surface is located at a proximal side of the groove; when the pusher is at an initial position, a portion of the pusher abuts against the initial surface to be at the first height.

For example, in the surgical instrument provided in an embodiment of the present disclosure, the pusher is further provided with a pressurized end extending in a direction away from the blade lifting portion, and the cartridge holder is further provided with a first elastic member; one end of the first elastic member is connected to the cartridge holder, and the other end abuts against the pressurized end, the first elastic member exerting a downward elastic force on the pressurized end; when the end effector is in the second state, in response to the pusher moving distally, the pusher moves downward under the action of the elastic force of the first elastic member to cause the limiting portion to enter the groove.

For example, in the surgical instrument provided in an embodiment of the present disclosure, a guide inclined surface is provided at the proximal side of the groove, and the guide inclined surface is connected to the initial surface.

For example, in the surgical instrument provided in an embodiment of the present disclosure, the pusher includes a cutter bar and a cutter head connected to the cutter bar, and the blade lifting portion is provided at a lower side of the cutter head; in response to a user's operation, the cutter bar drives the cutter head to move distally.

In the surgical instrument provided by the embodiments of the present disclosure, by providing a limiting structure on the cartridge holder for limiting and stopping the pusher, when the surgical instrument is mounted with a used cartridge assembly or no cartridge assembly at all, the pusher is limited and stopped to prevent the pusher from driving the pusher plate and the cutting blade to move distally, so that the surgical instrument cannot perform a cutting operation, thereby avoiding injury to the human body. In the embodiments of the present disclosure, by providing a support structure at the bottom of the pusher plate to lift the blade lifting portion of the pusher so that the pusher passes over the limiting structure and moves distally to a blade advancement position, when the surgical instrument is normally mounted with an unused cartridge assembly, the support structure of the pusher plate lifts the blade lifting portion, so that the pusher does not contact the limiting structure on the cartridge holder during its distal movement. Thus, the pusher can normally drive the pusher plate and the cutting blade to move distally, allowing the surgical instrument to perform suturing and cutting operations, ensuring normal use of the surgical instrument, and solving the problem that a surgical instrument with a cartridge integrated with a cutting blade has a risk of injuring the human body when a used cartridge assembly or no cartridge assembly is mounted.

### BRIEF DESCRIPTION OF DRAWINGS

Fig. 1 is an exploded view of a surgical instrument provided in an embodiment of the present disclosure;
Fig. 2 is a cross-sectional view of a surgical instrument provided in an embodiment of the present disclosure;
Fig. 3 is a schematic structural view of a pusher in an initial position provided in an embodiment of the present disclosure;
Fig. 4 is a schematic structural view of a pusher in a locked position provided in an embodiment of the present disclosure;
Fig. 5 is a schematic structural view of a pusher plate provided in an embodiment of the present disclosure;
Fig. 6 is a cross-sectional view of a pusher plate provided in an embodiment of the present disclosure; and
Fig. 7 is a schematic installation view of a cartridge assembly provided in an embodiment of the present disclosure.

### Reference Numerals

10: pusher; 11: cutter bar; 12: cutter head; 121: blade lifting portion; 1211: first abutting surface; 1212: second abutting surface; 1213: driving inclined surface; 1214: avoidance inclined surface; 122: support portion; 13: limiting portion; 14: pressurized end; 20: cartridge holder; 21: groove; 211: guide surface; 212: stopping wall; 213: guide inclined surface; 22: stopping protrusion; 23: first elastic member; 24: initial surface; 30: anvil; 40: cartridge assembly; 41: pusher plate; 411: support groove; 4111: inclined section; 412: side wall; 413: slide groove; 414: second elastic member; 415: blade retraction groove; 42: cutting blade; 421: guide protrusion; 43: cartridge body; and 431: staple ejection surface.

### DETAILED DESCRIPTION

To make the objectives, technical solutions and beneficial effects of the present disclosure more clear and explicit, the present disclosure is described in further detail below in conjunction with the accompanying drawings and embodiments. It should be understood that the embodiments described herein are merely used to explain the present disclosure and are not intended to limit the present disclosure. All other embodiments obtained by those of ordinary skill in the art based on the embodiments of the present disclosure without creative efforts shall fall within the protection scope of the present disclosure.

It should be noted that the terms "proximal side" (also referred to as the proximal end) and "distal side" (also referred to as the distal end) used herein are defined relative to the clinician operating the handle of the stapler. The term "proximal side" refers to the part close to the clinician, and the term "distal side" refers to the part away from the clinician. That is, the handle is the proximal part and the jaw assembly is the distal part. For example, the proximal end of a component refers to the end relatively close to the handle, and the distal end refers to the end relatively close to the jaw assembly. The terms "upper" and "lower" are referenced to the relative positions of the anvil and the cartridge holder of the jaw assembly; specifically, the anvil is at the "upper" position and the cartridge holder is at the "lower" position. However, the stapler can be used in many directions and positions, and thus these terms describing relative positional relationships are not restrictive and absolute.

In the embodiments of the present disclosure, unless otherwise explicitly specified and limited, the terms "connected" and "coupled" shall be construed in a broad sense. For example, they may refer to a fixed connection, a detachable connection, a movable connection, or integration as a single body; they may also refer to a direct connection, or an indirect connection via an intermediate medium, and may further refer to internal communication between two elements or an interaction relationship between two elements such as abutment. Those of ordinary skill in the art may understand the specific meanings of the above terms in the embodiments of the present disclosure according to specific circumstances. It should be noted that when the terms "connected" and "coupled" are preceded by a qualifying term, they shall have the meaning defined by the corresponding qualifying term, excluding only the obviously excluded situations and not excluding other possible situations. For example, a "detachable connection" refers to a connection that can be disassembled and does not include integration as a single body, but does not exclude a movable connection or the like.

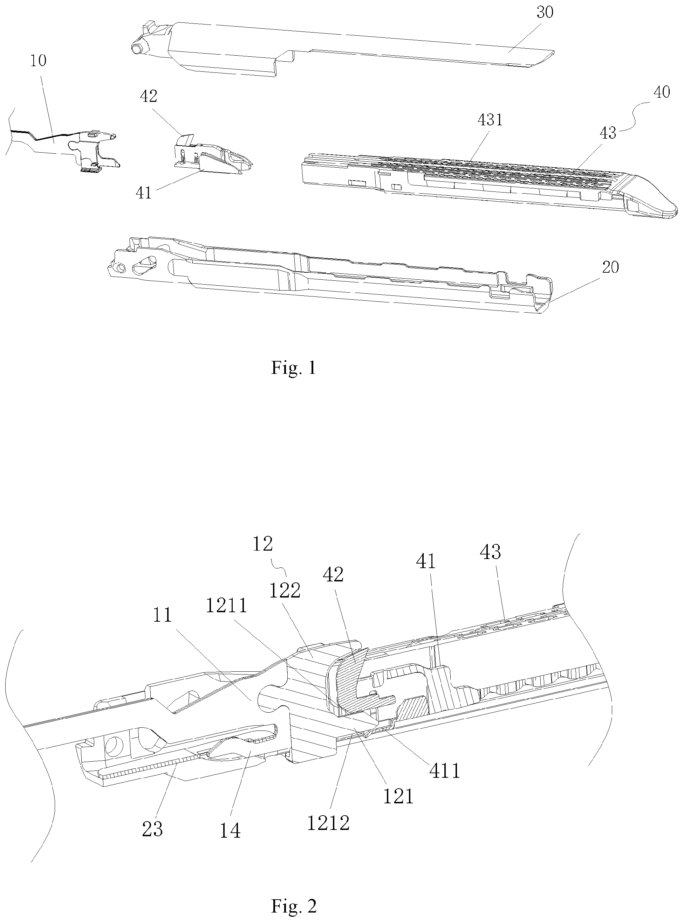

Refer to Figs. 1 to 7, a surgical instrument provided in an embodiment of the present disclosure includes an end effector, a sleeve assembly and a handle assembly, where the handle assembly is connected to the end effector via the sleeve assembly. The sleeve assembly includes a pusher 10 and a cannula sleeving a portion of the pusher 10. The surgical instrument further includes a transmission assembly and a power assembly, and the pusher 10 is drivingly connected to the power assembly via the transmission assembly, so as to be driven by the power assembly to move proximally or distally along the length direction of the cannula. In this embodiment, the power assembly includes a motor and a gear connected to a motor output shaft, and the transmission assembly includes a rack which meshes with the gear and is connected to the pusher. In response to the rotation of the motor, the gear rotates to drive the rack to move proximally or distally, thereby enabling the rack to drive the pusher proximally or distally. The end effector includes a jaw assembly and a cartridge assembly 40, the jaw assembly including a cartridge holder 20 and an anvil 30 pivotally connected to the cartridge holder 20. The cartridge assembly 40 is detachably mounted in the cartridge holder 20. The anvil 30 is selectively movable between an open position and a closed position to switch the end effector between an open state and a closed state, thus cooperating with the cartridge holder 20 and the cartridge assembly 40 to clamp or release tissue. The distal side of the cannula is connected to the anvil 30, and the proximal side is connected to the transmission assembly. Specifically, the transmission assembly further includes a jaw driving structure, and the proximal side of the cannula is connected to the jaw driving structure. In one embodiment, the jaw driving structure includes a jaw driving motor and a jaw driving assembly, the jaw driving assembly including a gear connected to the jaw driving motor and a rack meshing with the gear, with the rack connected to the cannula. In response to the rotation of the motor, the gear rotates to drive the rack to move, to drive the cannula to move forward or backward. In another embodiment, the jaw driving structure includes a handle, a connecting rod assembly connected to the handle, and an unlocking assembly, with the connecting rod assembly connected to the cannula. When the handle is pressed, the connecting rod assembly moves to drive the cannula to move forward; when the unlocking assembly is pressed, the unlocking assembly acts on the connecting rod to move the cannula backward. The backward (i.e., proximal) movement of the cannula causes the anvil 30 to pivot upward to open the jaw assembly, and the forward (i.e., distal) movement of the cannula causes the anvil 30 to pivot downward to close the jaw assembly. The cartridge assembly 40 includes a pusher plate assembly and a cartridge body 43, and the pusher plate assembly is removably connected to the pusher 10 to be driven by the pusher 10 to perform a firing action.

For example, the pusher plate assembly includes a pusher plate 41 and a cutting blade 42 movably connected to the pusher plate 41. The proximal side of the pusher 10 is connected to the transmission assembly, and the distal side is removably connected to the pusher plate 41 and the cutting blade 42 for driving the pusher plate 41 and the cutting blade 42 to move. That is to say, the surgical instrument in this embodiment is provided with a cartridge with an integrated cutting blade. The cartridge holder 20 is provided with a limiting structure for limiting and stopping the pusher 10. The pusher 10 is provided with a blade lifting portion 121 that extends distally, and the bottom of the pusher plate 41 is provided with a support structure. When the cartridge assembly 40 is mounted in the cartridge holder 20 and the pusher plate 41 is at a proximal position, the bottom of the blade lifting portion 121 is supported by the support structure, and the end effector is in a first state. In the first state, in response to a user's operation, the pusher 10 passes over the limiting structure and moves distally, and cooperates with the pusher plate 41 when moving distally to drive the pusher plate 41 to move distally; where the proximal position refers to the position where the pusher plate 41 is located at the proximal side of the cartridge assembly 40.

When no cartridge assembly 40 is mounted in the cartridge holder 20, or the pusher plate 41 of the mounted cartridge assembly 40 is not at the proximal side of the cartridge assembly 40 (in a fired cartridge assembly 40, the pusher plate 41 is located at the distal side of the cartridge assembly 40), the end effector is in a second state, and the blade lifting portion 121 is suspended within the cartridge holder 20. Suspension here means that the bottom of the blade lifting portion 121 is not supported by the support structure and is in a suspended state. In the second state, in response to a user's operation, the pusher 10 moves distally and is blocked by the limiting structure, so that the pusher 10 cannot continue to move distally and cannot perform firing normally.

In some embodiments of the present disclosure, when the cutting blade is not advanced, the pusher 10 is at a first height. When the end effector is in the first state, the support structure supports the pusher 10 at the bottom, so that the pusher 10 remains at the first height when moving distally, which can pass over the limiting structure to drive the pusher plate 41 to move distally, and can perform firing normally. When the end effector is in the second state, the pusher 10 is at the first height and the blade lifting portion 121 is suspended within the cartridge holder 20. In response to a user's operation, when the pusher 10 moves distally, it switches from the first height to a second height and then continues to move distally due to the lack of support for the blade lifting portion 121, and is blocked by the limiting structure. Where the second height is lower than the first height, and the limiting structure can only block the pusher 10 moving distally at the second height, but cannot block the pusher 10 moving distally at the first height. It can be seen from the above that the surgical instrument in this embodiment can only fire normally when the cartridge assembly 40 is mounted in the cartridge holder 20 and the pusher plate 41 is at the proximal side of the cartridge assembly 40, and cannot fire when no cartridge assembly 40 is mounted in the cartridge holder 20 or the pusher plate 41 of the mounted cartridge assembly 40 is not at the proximal side of the cartridge assembly 40. This avoids the problem that when a surgical instrument with a cartridge integrated with a cutting blade is mounted with a used cartridge assembly 40 or no cartridge assembly 40 at all, the pusher 10 moving distally directly compresses the tissue instead of driving the cutting blade 42 to cut the tissue distally, causing injury to the tissue.

The support structure includes a support groove 411 formed at the bottom of the pusher plate 41, and the support groove 411 includes a bottom wall. When the end effector is in the first state, the bottom wall of the support groove 411 abuts against the blade lifting portion 121 to support the blade lifting portion 121, and at this time, the blade lifting portion 121 is partially located in the support groove 411; when the pusher 10 moves distally, the lower end of the blade lifting portion 121 abuts against the bottom wall of the support groove 411 and slides relative to the support groove 411. After the blade lifting portion 121 completely enters the support groove 411, the pusher 10 still remains at the first height under the support of the support groove 411 and can drive the pusher plate 41 to move distally for firing. The abutment of the support groove 411 supporting the blade lifting portion 121 prevents the pusher 10 from moving downward to the second height, and the pusher 10 at the first height can smoothly pass over the limiting structure to drive the pusher plate 41.

When mounting the cartridge assembly 40 with the pusher plate 41 at the proximal side, after the cartridge assembly 40 is inserted into the cartridge holder 20, the bottom wall of the support groove 411 extends into the lower side of the blade lifting portion 121 and supports the blade lifting portion 121. To facilitate the mounting of the cartridge assembly 40, the support groove 411 further includes an inclined section 4111 which is located at the proximal side of the bottom wall and connected to the bottom wall, with the proximal side of the inclined section 4111 being lower and the distal side being higher. That is, the inclined section 4111 is inclined downward in the proximal direction, and the cartridge assembly 40 is mounted from the distal side to the proximal side. Meanwhile, the lower side of the blade lifting portion 121 is provided with an avoidance inclined surface 1214. The arrangement of the inclined section 4111 and the avoidance inclined surface 1214 enables the inclined section 4111 to easily enter the space below the blade lifting portion 121 to guide the mounting of the cartridge assembly 40.

The pusher plate 41 is provided with a receiving groove in which the cutting blade 42 is accommodated, and the support groove 411 is located at the lower side of the receiving groove and is in communication with the receiving groove. Specifically, the pusher plate 41 has two oppositely arranged side walls 412, and at least a portion of the cutting blade 42 is accommodated between the two side walls 412. That is to say, the receiving groove is formed between the two side walls 412, and the cutting blade is slidable up and down in the receiving groove. Before the cartridge assembly 40 is mounted, the cutting blade 42 is completely located in the receiving groove and is in a blade retracted state. The side walls 412 are provided with a slide groove 413 that extends vertically, and the cutting blade 42 is provided with a guide protrusion 421 adapted to the slide groove 413. When extending, the cutting blade 42 extends vertically upward along the slide groove 413 via the guide protrusion 421, and a gap is provided between the cutting blade 42 and the side walls 412 to reduce the friction between the side walls 412 and the cutting blade 42. Meanwhile, the arrangement of the slide groove 413 stabilizes the extending direction of the cutting blade 42. The support groove 411 is located below the receiving groove and is open at the proximal side, enabling the blade lifting portion 121 to enter the support groove 411 from the proximal side. As a whole, the proximal side of the support groove 411 is trumpet-shaped.

The blade lifting portion 121 is provided with a driving inclined surface 1213 which is arranged on the upper side of the distal end of the blade lifting portion 121. When the cartridge assembly 40 is mounted, the lower side of the blade lifting portion 121 enters the support groove 411 and the upper side enters the receiving groove, and abuts against the cutting blade 42 via the driving inclined surface 1213. Under the action of the driving inclined surface 1213, the cutting blade 42 slides upward and is exposed from the receiving groove, switching the cutting blade 42 to a blade extended state. After the cartridge assembly 40 is mounted, the end effector is in the first state. At this time, a portion of the blade lifting portion 121 is received in the receiving groove and another part is received in the support groove 411; the upper end surface of the blade lifting portion 121 abuts against the cutting blade 42, the lower end surface abuts against the bottom wall of the support groove 411, and the blade lifting portion 121 does not abut against the distal side surface of the support groove 411, with the cutting blade 42 kept in the blade extended state.

In the first state, the pusher 10 cannot directly drive the pusher plate 41 to move when moving distally. When the pusher 10 moves distally, it slides relative to the pusher plate 41 until the pusher 10 cooperates with the pusher plate 41. Cooperation here means that the blade lifting portion 121 abuts against the distal side surface of the support groove 411, and at this time, the pusher 10 can drive the pusher plate 41 to move distally for firing. During the process that the blade lifting portion 121 moves distally to be engaged with the pusher plate 41, the pusher 10 remains at the first height under the action of the support groove 411 and can pass over the limiting structure without being blocked by it.

The pusher 10 is provided with a limiting portion 13 which is used to be blocked by the limiting structure when the pusher 10 is at the second height. When the pusher 10 moves distally at the first height, the limiting portion 13 is higher than the limiting structure to enable the pusher 10 to pass over the limiting structure; when the pusher 10 moves distally at the second height, the limiting structure blocks the limiting portion 13 to block the pusher 10.

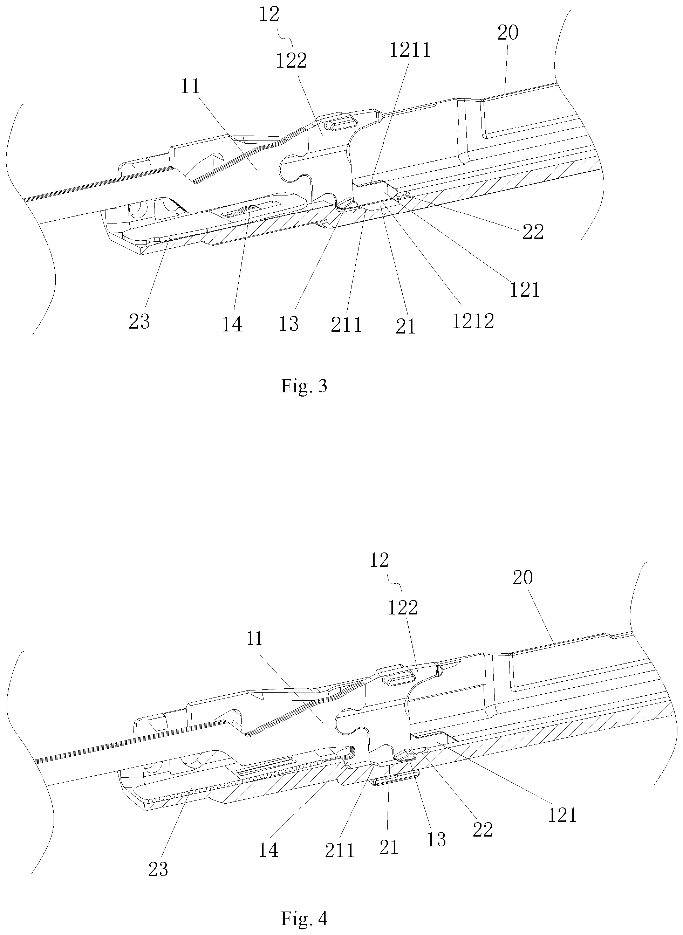

Refer to Figs. 3 to 4, in this embodiment, the limiting structure is a groove 21, and the limiting portion 13 is a protrusion. The groove 21 includes a stopping wall 212 which is located at the distal side of the groove 21. The limiting portion 13 is arranged at the side of the pusher 10. When the pusher 10 is at the first height, the limiting portion 13 is higher than the upper edge of the stopping wall 212 and located outside the groove 21, so the stopping wall 212 cannot block the limiting portion 13 when the pusher 10 moves distally at the first height; when the pusher 10 is at the second height, the limiting portion 13 is lower than the upper edge of the stopping wall 212 and located inside the groove 21, so the stopping wall 212 blocks the limiting portion 13 when the pusher 10 moves distally at the first height, preventing the pusher 10 from continuing to move distally. Furthermore, refer to Figs. 3 to 4, the cartridge holder 20 further includes a stopping protrusion 22 which is located at the distal side of the stopping wall 212 and higher than the upper edge of the stopping wall 212. This arrangement can enhance the limiting effect of the limiting structure and prevent the pusher 10 moving distally at the second height from passing over the limiting structure due to a relatively high moving speed.

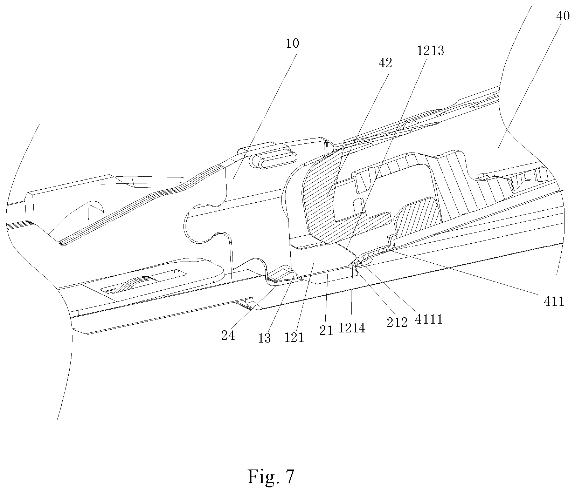

Refer to Fig. 7, the cartridge holder 20 further includes an initial surface 24, which is located at the proximal side of the groove 21. When the pusher 10 is at the initial position, a portion of the pusher 10 abuts against the initial surface 24 to be maintained at the first height. The blade lifting portion 121 is located at the distal side of the initial surface 24 and suspended above the groove 21. Specifically, when the pusher 10 is at the initial position, the limiting portion 13 abuts against the initial surface 24, so that the limiting portion 13 is located at the proximal side of the groove 21 and above the groove 21.

When no cartridge assembly 40 is mounted in the cartridge holder 20, or the pusher plate 41 of the mounted cartridge assembly 40 is not at the proximal side of the cartridge assembly 40, the blade lifting portion 121 is unsupported. When the pusher 10 moves distally from the initial position, it first moves downward into the groove 21, and the limiting portion 13 abuts against the bottom surface of the groove 21, so that the pusher 10 is maintained at the second height.

To ensure that the pusher 10 can stably enter the groove 21 without directly passing over the limiting structure when the blade lifting portion 121 is unsupported, the pusher 10 is further provided with a pressurized end 14 extending in a direction away from the blade lifting portion 121, and the cartridge holder 20 is further provided with a first elastic member 23. One end of the first elastic member 23 is connected to the cartridge holder 20, and the other end abuts against the pressurized end 14 and exerts a downward elastic force on the pressurized end 14. The pusher 10 is at the initial position before moving distally, where the limiting portion 13 is located above the groove 21. When the pusher 10 moves distally from the initial position, it moves downward under the action of the elastic force of the first elastic member 23, so that the limiting portion 13 enters the groove 21. In this embodiment, the first elastic member 23 is a leaf spring. The proximal side of the leaf spring is fixed to the cartridge holder 20, and the distal side presses the pressurized end 14 against the bottom of the cartridge holder 20, and the distal side of the leaf spring is provided with an intermediate through-hole at the proximal protrusion of the pressurized end 14 to improve the fixing effect. Of course, the intermediate through-hole has a proper length so as not to affect the normal movement of the pusher 10. When the surgical instrument is fitted with a used cartridge assembly 40 or no cartridge assembly 40 at all, the leaf spring constantly presses against the pressurized end 14, and there is no pusher plate 41 at the proximal side of the cartridge holder 20, so that the blade lifting portion 121 cannot switch to the engaged state. As a result, the limiting portion 13 enters the groove 21 and is limited and stopped by the stopping wall of the groove 21 during the distal movement of the pusher 10. When the surgical instrument is normally mounted with an unused cartridge assembly 40, the leaf spring also constantly presses against the pressurized end 14, but the blade lifting portion 121 switches to the engaged state to lift the pusher 10 against the elastic force of the leaf spring, so that the leaf spring does not affect the distal movement of the pusher 10.

It should be noted that a guide inclined surface 213 is provided at the proximal side of the groove 21, and the guide inclined surface 213 is connected to the initial surface 24. After the pusher 10 moves distally to drive the pusher plate 41 to complete the distal firing, the pusher 10 moves proximally back to the initial position in response to a user's operation. When the pusher 10 moves proximally, the limiting portion 13 is attached to the bottom wall of the cartridge holder 20. When passing over the groove 21, the limiting portion 13 enters the groove 21, abuts against the bottom surface of the groove 21, and moves proximally inside the groove 21. When the limiting portion 13 comes into contact with the guide inclined surface 213, the guide inclined surface 213 guides the limiting portion 13 to make the pusher 10 move upward passing over the groove 21, and the limiting portion 13 abuts against the initial surface 24, so that the pusher 10 returns to the initial position smoothly. Specifically, refer to Figs. 2 to 4, the pusher 10 includes a cutter bar 11 and a cutter head 12. The cutter head 12 is connected to the distal side of the cutter bar 11, and the proximal side of the cutter bar 11 is connected to the transmission assembly. The limiting portion 13 is arranged on one side of the cutter head 12 and at the side of the cutter head close to the bottom of the cartridge holder 20. When the cartridge assembly 40 is mounted in the cartridge holder 20 and the pusher 10 moves distally along the length direction, the cutter head 12 pushes the pusher plate 41 to move forward, and the cutter head 12 contacts and cooperates with the cutting blade 42, so that the cutting blade 42 is lifted up and further removably connected to the cutter head 12, with the cutting edge of the cutting blade 42 protruding from the staple ejection surface 431 of the cartridge body 43. Furthermore, the cutter head 12 includes a blade lifting portion 121 and a support portion 122. The blade lifting portion 121 is located at the lower side of the cutter head 12, and the support portion 122 is located at the upper side of the cutter head 12, with the blade lifting portion 121 and the support portion 122 arranged in a corresponding position.

After passing over the limiting structure, the pusher 10 drives the pusher plate 41 to move distally for the advancement of the cutting blade. The advancement of the cutting blade is completed when the pusher plate 41 moves to the distal side of the cartridge assembly 40. At this time, the pusher 10 moves proximally for the retraction of the cutting blade. The pusher 10 can only drive the pusher plate 41 to move distally and cannot pull the pusher plate 41 to move proximally. The blade lifting portion 121 moves proximally with the pusher 10 and is separated from the pusher plate 41. The pusher 10 moves proximally back to the initial position, while the pusher plate 41 remains at the distal side of the cartridge assembly 40.

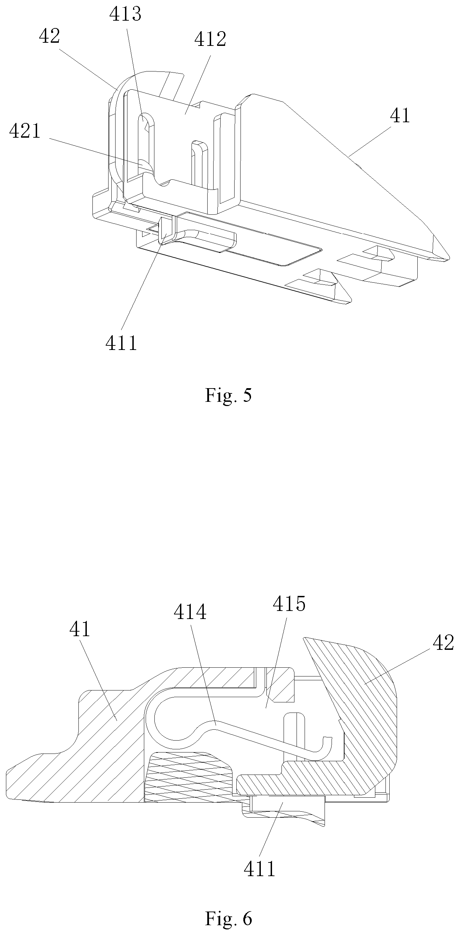

Specifically, refer to Fig. 6, the pusher plate 41 is provided with a blade retraction groove 415 capable of accommodating at least a portion of the cutting blade 42, and a second elastic member 414 is further arranged in the pusher plate 41 and received in the blade retraction groove 415. Specifically, the second elastic member 414 is a special-shaped spring. One end of the second elastic member 414 abuts against the cutting blade 42, and the other end abuts against the inner wall of the blade retraction groove 415, which is used to exert an elastic force on the cutting blade 42 to keep the cutting blade 42 in a retracted state. When the driving inclined surface 1213 of the blade lifting portion 121 contacts the bottom of the cutting blade 42, the cutting blade 42 moves upward, the second elastic member 414 is compressed, and the cutting blade 42 continues to move upward and extend out, switching from the retracted state to the blade extended state. When the pusher 10 carries the blade lifting portion 121 to move proximally during the retraction of the cutting blade and the blade lifting portion 121 no longer abuts against the cutting blade 42, the second elastic member 414 rebounds to drive the cutting blade 42 to retract into the blade retraction groove 415.

After the pusher 10 returns to the initial position, the retraction of the cutting blade is completed. The medical staff controls the jaw assembly to open and moves the stapler out of the human body, and the surgery is completed.

When the surgical instrument provided in the embodiments of the present disclosure is mounted with a used cartridge assembly 40 or no cartridge assembly 40 at all, the end effector is in the second state. At this time, the pusher 10 is blocked by the limiting structure and cannot continue to move when moving distally, which avoids the injury to human tissue caused by a direct compression of the tissue by the pusher 10. Only when the cartridge assembly 40 is mounted in the cartridge holder 20 and the pusher plate 41 is at the proximal position, the end effector is in the first state, lifting the pusher 10 to pass over the limiting structure and move distally for the advancement of the cutting blade. This ensures the normal use of the surgical instrument and avoids the risk of injury to the human body when a surgical instrument with a cartridge integrated with a cutting blade is mounted with a used cartridge assembly 40 or no cartridge assembly 40 at all.

It should be understood that although the present specification is described in terms of embodiments, each embodiment does not only include an independent technical solution. The narrative manner of the specification is only for the sake of clarity, and those skilled in the art shall regard the specification as a whole. The technical solutions in the various embodiments may also be appropriately combined to form other embodiments understandable to those skilled in the art.

The series of detailed descriptions listed above are only specific illustrations of the feasible embodiments of the present disclosure, and they are not intended to limit the protection scope of the present disclosure. All equivalent embodiments or modifications made without departing from the technical spirit of the present disclosure shall be included in the protection scope of the present disclosure.

## Claims

1. A surgical instrument, comprising an end effector and a sleeve assembly, wherein the sleeve assembly comprises a pusher,
the end effector comprises a jaw assembly and a cartridge assembly, wherein the jaw assembly comprises a cartridge holder and an anvil pivotally connected to the cartridge holder, a limiting structure is provided at the cartridge holder, the cartridge assembly is detachably mounted in the cartridge holder, and the cartridge assembly comprises a pusher plate and a cutting blade movably connected to the pusher plate;
a blade lifting portion is provided at a bottom of the pusher, a support structure is provided at a bottom of the pusher plate, wherein when the cartridge assembly is mounted in the cartridge holder and the pusher plate is at a proximal position, the blade lifting portion is supported by the support structure, and the end effector is in a first state;
when the end effector is in the first state, the pusher, in response to a user's operation, passes over the limiting structure and moves distally to be engaged with the pusher plate so as to drive the pusher plate to move distally;
when the cartridge assembly is not mounted in the cartridge holder or the pusher plate is at a distal position, the blade lifting portion is suspended within the cartridge holder, and the end effector is in a second state; and
when the end effector is in the second state, the pusher, in response to the user's operation, is blocked by the limiting structure when moving distally.

2. The surgical instrument according to claim 1, wherein the support structure comprises a support groove formed at the bottom of the pusher plate, and the support groove comprises a bottom wall,
wherein when the end effector is in the first state, the bottom wall of the support groove abuts against the blade lifting portion to support the blade lifting portion.

3. The surgical instrument according to claim 2, wherein the support groove further comprises an inclined section, wherein the inclined section is located at a proximal side of the bottom wall and connected to the bottom wall, and a height of a distal side of the inclined section is higher than a height of a proximal side of the inclined section.

4. The surgical instrument according to claim 2, wherein the pusher plate is provided with a receiving groove, the cutting blade is received in the receiving groove, and the receiving groove is in communication with the support groove,
wherein when the end effector is in the first state, a portion of the blade lifting portion is received in the receiving groove and another portion of the blade lifting portion is received in the support groove; and
an upper end surface of the blade lifting portion abuts against the cutting blade to keep the cutting blade in a blade extended state, and a lower end surface of the blade lifting portion abuts against the bottom wall of the support groove.

5. The surgical instrument according to claim 1, wherein the pusher comprises a limiting portion,
wherein when the end effector is in the first state, the limiting portion is at a first height and the limiting portion is higher than the limiting structure to cause the pusher to pass over the limiting structure; and
when the end effector is in the second state, the limiting portion is at a second height, and the limiting structure blocks the limiting portion to block the pusher, wherein the second height is lower than the first height.

6. The surgical instrument according to claim 5, wherein the limiting structure is a groove, and the groove comprises a stopping wall,
wherein when the pusher is at the first height, the limiting portion is higher than an upper edge of the stopping wall and the limiting portion is located outside the groove; and
when the pusher is at the second height, the limiting portion is lower than the upper edge of the stopping wall and the limiting portion is located inside the groove.

7. The surgical instrument according to claim 6, wherein the cartridge holder further comprises a stopping protrusion, and the stopping protrusion is located at a distal side of the stopping wall and the stopping protrusion is higher than the upper edge of the stopping wall.

8. The surgical instrument according to claim 6, wherein the cartridge holder further comprises an initial surface, and the initial surface is located at a proximal side of the groove, and
when the pusher is at an initial position, a portion of the pusher abuts against the initial surface to be at the first height.

9. The surgical instrument according to claim 8, wherein the pusher is further provided with a pressurized end extending in a direction away from the blade lifting portion, and the cartridge holder is further provided with a first elastic member,
wherein one end of the first elastic member is connected to the cartridge holder, and another end abuts against the pressurized end to exert an elastic force towards the bottom of the cartridge holder to the pressurized end; and
when the end effector is in the second state, the pusher, in response to the pusher moving distally, moves towards the bottom of the cartridge holder under the action of the elastic force of the first elastic member to cause the limiting portion to enter the groove.

10. The surgical instrument according to claim 8, wherein a guide inclined surface is provided at the proximal side of the groove, and the guide inclined surface is connected to the initial surface.

11. The surgical instrument according to claim 1, wherein the pusher comprises a cutter bar and a cutter head connected to the cutter bar, the blade lifting portion is provided at a side of the cutter head close to the bottom of the cartridge holder,
wherein the cutter bar is configured to, in response to a user's operation, drive the cutter head to move distally.
